# EUROPEAN PATENT APPLICATION

(11) **EP 1 440 699 A1**
(43) Date of publication of application: **28.07.2004**
(21) Application number: 04001100.9
(22) Date of filing: 20.01.2004
(51) Int. Cl.: A61L 31/10, A61L 31/16

(54) **Stent with epoxy primer coating**

(30) Priority: 24.01.2003 US 350787
(71) Applicant: Medtronic Vascular, Inc., Santa Rosa, California 95403 (US)
(72) Inventor: Cheng, Peiwen, Santa Rosa, California 95409 (US); Sundar, Ranjarajan, Santa Rosa, California 95404 (US); Kaushik, Patel, wINDSOR, California 95492 (US); Upidi, Kishore, Santa Rosa, California 95403 (US)
(74) Representative: Hughes, Andrea Michelle

(57) **Abstract**

The present invention provides a system for treating a vascular condition, comprising a catheter; a stent coupled to the catheter, the stent including a stent framework; an epoxy primer coating disposed on the stent framework; and a drug-polymer coating disposed on the epoxy primer coating. The present invention also provides an epoxy-coated stent, a drug-coated stent with an epoxy primer coating, and a method of manufacturing the coated stents.

## Description

### FIELD OF THE INVENTION

This invention relates generally to biomedical stents and catheters with stents. More specifically, the invention relates to a primer for a stent that may be subsequently coated with a drug polymer.

### BACKGROUND OF THE INVENTION

Biomedical stents are typically formed from metallic or polymeric materials, and deployed in the body to reinforce blood vessels and other vessels within the body as part of surgical procedures that require enlargement and stabilization of the lumens. Endovascular stents may be coated with polymeric coatings to prevent corrosion of the stent material within the body. They may also be coated with drug-polymers that contain one or more therapeutic compounds within a polymeric matrix to improve the efficacy of the deployed stents. These compounds are eluted from the stent coating to the tissue bed surrounding the implanted stent. The effectiveness of these drugs is generally improved because localized levels of medication can be higher and potentially more potant than orally or intravenously delivered drugs, which are distributed throughout the body rather than concentrated at the location of most need. Drugs released from tailored stent coatings may have controlled, timed-release qualities, eluting their bioactive agents over hours, weeks or even months. The drug-polymer coating may be applied over the stent framework. Typically, a common solvent or a pair of solvents is used to dissolve the drugs and selected polymers such as copolymers, terpolymers or polymer blends. Then the drug-polymer solution is sprayed or dipped on the stent. Upon drying, the drug-polymer coating is formed on the stent surface.

Polymer matrices containing the compounds must be reliably attached to the stent to control delivery of the pharmaceutical compounds, to maintain high quality during manufacturing of such a stent, and to prevent cracking or flaking of the drug-polymer coating when the stent is deployed. There have been problems in getting coatings to adhere to stents, particularly stents made of stainless steel and other metals. Most coronary stents are made of stainless steel or tantalum and are finished by electrochemical polishing for surface smoothness. A smooth surface is desirable because early research has shown that a stent with a rough surface results in more platelet cell adhesion, thrombus, inflammation, and restenosis than when a stent is smoothly polished. The smooth surface may pose a challenge to the coating, however. Due to the very different nature of the polymer and the metallic substrate, polymers do not easily adhere to the metallic substrate. If the coating does not adhere well to the metal surface, it may cause problems such as coating delamination, irregular drug release profiles, or embolism as a result of broken and detached debris from the coating.

The coating may crack or fall off during assembly, packaging, storage, shipping, preparation and sterilization prior to deployment unless it is effectively adhered to the stent framework. Additionally, degradation of the polymer coating may occur with prolonged exposure to light and air, as constituents of the drug polymer oxidize or the molecular chains scission. Although degradation of the polymer coating is of major concern, it is imperative that the adhesion strength of the coating be greater than the cohesive strength of the polymeric matrix to avoid any loss of the coating.

Polymeric coatings have a tendency to peel or separate from an underlying metallic stent because of the low adhesion strength typically found between polymers and metals. Many polymers are non-polar or have limited polarization, reducing their ability to stick to the metal stent framework. Temperature excursions of the coated stent and the difference in thermal expansion coefficients between the metal and the coating may contribute to the fatigue and failure of the bond. Materials that are optimal for drug compatibility and elution may not, in and of themselves, provide sufficient adhesion to the framework of a metallic or polymeric stent. A method to improve the adhesion between a drug-polymer coating and a stent, while retaining the therapeutic characteristics of the drug-polymer stent, would be beneficial. In an improved method, conventional polymers would be readily incorporated into the drug-polymer coating, provided the stent framework is adapted to provide improved adhesion. If the adhesive strength of the polymeric coating is improved, a more robust stenting device could be made. The coating profiles also could be thinner, and the stent struts could touch. It is desirable to have an adhesion layer or primer on the stent framework that is biocompatible, promotes good adhesion between metals and subsequently applied polymers, is easy to process, and is reliable. The primer coating should not soften, delaminate, or dissolve in the solvent used for the topcoat.

It is an object of this invention, therefore, to provide a flexible, durable, chemical-resistant coating for a metallic stent to protect the stent from degradation within the body. It is another objective of this invention to provide a drug-coated stent with an effective adhesion layer between the drug polymer and the underlying stent framework. It is another objective to provide a method for manufacturing a coated metallic stent, and for manufacturing a drug-polymer coated stent with an effective adhesion coating or primer. It is another objective of this invention to provide a system for treating heart disease and other vascular conditions using drug-eluting stents with improved adhesion between the drug polymer and the stent framework, and to overcome the deficiencies and limitations described above.

### SUMMARY OF THE INVENTION

One aspect of the present invention provides a coated stent, comprising:
a stent framework; and
an epoxy coating disposed on the stent framework.

Another aspect provides a drug-coated stent, comprising:
a stent framework;
an epoxy primer coating disposed on the stent framework; and
a drug-polymer coating disposed on the epoxy primer coating.

There is also provided a system for treating a vascular condition that includes a catheter; and such a stent coupled to the catheter. The catheter may include a balloon used to expand the stent and a sheath that retracts to allow the expansion of the stent.

Another aspect of the present invention provides a method of manufacturing a coated stent. An epoxy resin is mixed with a solvent to form an epoxy resin solution. A cross-linking agent may be added to the epoxy resin solution. The epoxy resin solution is applied onto the stent framework, and the epoxy resin is cured. A drug-polymer coating may be applied to the cured epoxy resin solution disposed on the stent framework.

### BRIEF DESCRIPTION OF THE DRAWINGS

The present invention is illustrated by the accompanying drawings of various embodiments and the detailed description given below. The detailed description and drawings are merely illustrative of the preferred embodiments of the invention amd are given by way of example only. The foregoing aspects and other attendant advantages of the present invention will become more readily appreciated by the detailed description taken in conjunction with the accompanying drawings, wherein:
FIG. 1 illustrates one embodiment of a system for treating a vascular condition including a catheter, a stent, an epoxy primer coating, and a drug-polymer coating, in accordance with the current invention;
FIG. 2 illustrates a stent cross-section with an epoxy coating on the stent surface, in accordance with the current invention;
FIG. 3 illustrates a cross section of a drug-coated stent with an epoxy primer coating between a drug-polymer coating and the stent framework, in accordance with the current invention; and
FIG. 4 is a flow diagram of one embodiment of a method for manufacturing a coated stent or a drug-coated stent with an underlying epoxy primer coating, in accordance with the current invention.

### DETAILED DESCRIPTION OF THE

### PRESENTLY PREFERRED EMBODIMENTS

In one embodiment, the present invention provides an epoxy-based barrier coating for an endovascular stent that is flexible, durable, and chemical resistant. In another embodiment, the invention includes an epoxy primer coating used to coat a metallic or polymeric stent that serves as an adhesion layer between a drug-polymer coating and the underlying stent framework.

Polymer coatings based on cross-linked epoxy resins may be used as effective coatings on metallic stents to reduce the potential for injury to the bodily vessels during insertion and to reduce the possibility of corrosion during prolonged usage within the body. A polymer coating based on epoxy resin may also be employed as a primer coating to promote adhesion between a metal stent surface and a subsequent polymer coating such as a drug-polymer coating. A drug polymer may be applied to a stent after an epoxy primer coating has been applied and cured. The subsequent polymer coating may contain one or more therapeutic compounds to provide pharmaceutical properties to the drug-coated stent. The primer coating acts as a bridge between substrates and organic polymer coatings, with good adhesion properties to both the metal and the drug polymer.

The epoxy resins are high molecular weight, linear polymers derived from suitable sources of the backbone group such as bisphenol-A and epichlorohydrin. Epoxy coatings containing epoxy resins have a unique combination of toughness and flexibility. Epoxy resins comprise oxirane groups that can readily participate in cross-linking reactions. A wide variety of cross-linkers, also known as curing agents, have been found useful in obtaining three-dimensional, insoluble and infusible yet highly flexible networks. Such cross-linkers include polyamines, polyamides, polyacids, polyanhydrides, and other epoxy curing agents.

The degree of cross-linking affects the flexibility and the chemical inertness of the epoxy coating. A higher level of cross-linking, for example, results in a tightly cross-linked network with less flexibility and generally higher chemical resistance. A lower level of cross-linking, on the other hand, may result in a more flexible coating, though with higher chemical sensitivity. An effective amount of cross-linking results in a stent coating that is flexible enough to meet the requirements of stent deployment, yet is able to provide sufficient chemical resistance for the stent framework when deployed within the body.

Another aspect of the present invention is a system for treating coronary heart disease and other vascular conditions, using catheter-deployed endovascular stents with polymeric coatings that include one or more drugs with desired timed-release properties and an epoxy primer coating that serves as an adhesion promoter or an adhesion layer between the stent framework and the drug polymer. Treatment of vascular conditions may include the prevention or correction of various ailments and deficiencies associated with the cardiovascular system, urinogenital systems, biliary conduits, abdominal passageways and other biological vessels within the body.

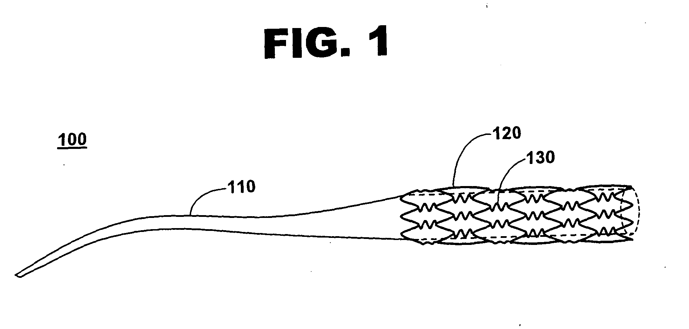

One embodiment of a system for treating a vascular condition, in accordance with the present invention, is illustrated in FIG. 1 at 100. Vascular condition treatment system 100 may include a catheter 110, a stent 120 coupled to catheter 110, and a drug-polymer coating 130 with an underlying epoxy primer coating on the stent framework.

Stent 120 is coupled to catheter 110, and may be deployed, for example, by pressurizing a balloon coupled to the stent or by retracting a sheath that allows the stent to expand to a prescribed diameter. Stent 120 includes a stent framework. The stent framework may be formed from a metallic base such as stainless steel, nitinol, tantalum, MP35N alloy, platinum, titanium, a suitable biocompatible alloy, or other suitable metal alloy. The stent framework may be formed from a polymeric base.

The epoxy primer coating may be disposed on the stent framework. The epoxy primer coating may comprise, for example, an epoxy resin or an epoxy polymer. The epoxy resin has amine groups that link to the underlying stent material to improve the adhesion between the epoxy primer coating and the stent framework. The oxirane linkages and pendant hydroxyl groups improve wettability and bonding to the stent material and to overcoated polymeric materials.

A drug polymer may be disposed on the stent framework. The drug polymer may be applied to the stent after the epoxy primer coating is disposed on the stent framework. The adhesion of the drug polymer to a stent coated with a polymeric primer layer or coating may be enhanced because the drug polymer is essentially coating over similar material.

Drug-polymer coating 130 includes one or more drugs. Each drug may include a bioactive agent. The bioactive agent may be a pharmacologically active drug or bioactive compound. The bioactive agent may be eluted from the drug-polymer coating when the stent has been deployed in the body. Elution refers to the transfer of the bioactive agent out from drug-polymer coating 130. The elution rate is determined by the rate at which the bioactive agent is excreted from drug-polymer coating 130 into the body. The composition of the drug-polymer coating and the interdispersed drugs may control the elution rate of the bioactive agent. The epoxy primer coating underlying drug-polymer coating 130 tends not to be eluted, metabolized, or discarded by the body.

The drug-polymer coating may be subject to degradation during processing, packaging, sterilization, or storage of a drug-polymer coated stent. During sterilization, for example, oxidation of the drug or polymer may occur, resulting in hydrolytic damage, cleavage of the polymeric bonds, breakdown of the polymer and/or drug, or actual cracking or peeling of the drug-polymer coating. Temperature excursions of the in-process or processed stent may incite delamination of all or a portion of the drug-polymer coating. The present invention solves this problem through the use of an epoxy primer coating between the polymer-drug coating and the metallic stent, so as to reduce or prevent drug-polymer delamination.

Upon insertion of catheter 110 and stent 120 with drug-polymer coating 130 into a directed vascular region of a human body, stent 120 may be expanded by applying pressure to a suitable balloon inside the stent, or by retracting a sheath to allow expansion of a self-expanding stent. Balloon deployment of stents and self-expanding stents are well known in the art. Catheter 110 may include a balloon used to expand stent 120. Alternatively, catheter 110 may include a sheath that retracts to allow expansion of the stent.

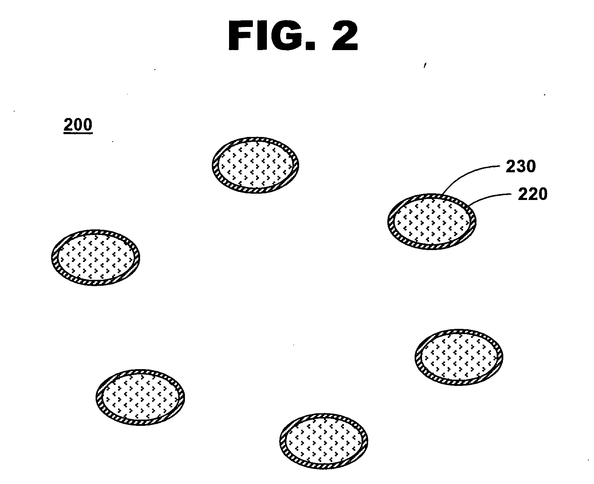

FIG. 2 shows an illustration of a stent cross-section including an epoxy coating on the stent surface, in accordance with the present invention at 200. Epoxy-coated stent 200 with may include a stent framework 220 with an epoxy coating 230 on stent framework 220. Epoxy coating 230 may contain long chains of epoxy resin or any suitable form of epoxy polymer. The molecular weight of epoxy coating 230 may be between 1,000 and 10,000 or more. Epoxy coating 230 may contain a polymeric matrix with interdispersed epoxy resin polymers. Epoxy coating 230 may include one or more cross-linking agents. A drug-polymer coating may be disposed on top of epoxy coating 230.

Stent framework 220 is typically composed of a metallic or polymeric base. Stent framework 220 may include a base material such as stainless steel, nitinol, tantalum, MP35N alloy, platinum, or titanium. The stent or stent framework may include a base material of a suitable biocompatible alloy, a suitable biocompatible material including a biodegradable polymeric material, or a combination thereof.

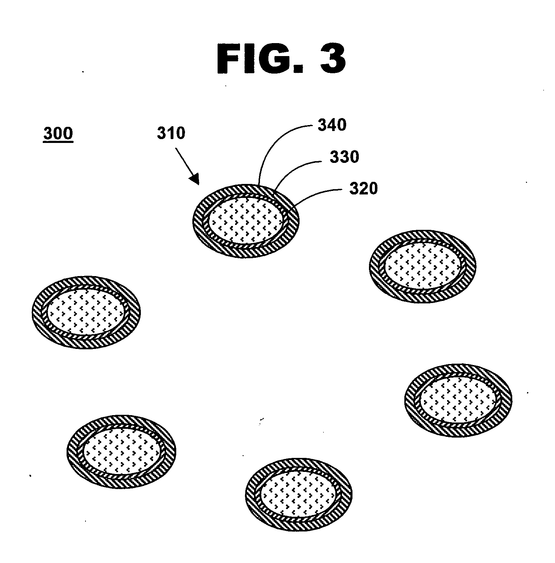

FIG. 3 shows an illustration of a stent cross section comprised of a polymeric coating containing a drug-polymer coating disposed on an epoxy primer coating between the drug-polymer coating and the stent framework, in accordance with another embodiment of the present invention at 300. Drug- coated stent 300 with polymeric coating 310 includes an epoxy primer coating 330 on a stent framework 320 and a drug-polymer coating 340 on epoxy primer coating 330. Epoxy primer coating 330 may be referred to herein as an adhesive coating. Drug-polymer coating 340 includes at least one bioactive agent. Epoxy primer coating 330 may be void or nearly void of pharmaceutical drugs. Epoxy primer coating 330 may include one or more cross-linking agents.

Epoxy primer coating 330 may be selected to improve the adhesion and minimize the likelihood of delamination of the polymeric coating from stent framework 320. Metal-adhering attributes of the primer layer aid in the cohesiveness of the polymeric coating to metallic stents. Stent framework 320 may comprise a metallic or a polymeric base.

Epoxy primer coating 330 may be comprised of an epoxy resin or epoxy polymeric material that enhances adhesion between drug-polymer coating 340 and stent framework 320. Epoxy primer coating 330 may comprise a series of long-chain polymers with predominantly epoxy groups along the backbone. The linear polymeric chains may be cross-linked to varying degrees based on the amount of cross-linking agents and the degree of curing. Various pharmaceutical compounds may be contained within drug-polymer coating 340.

The pharmaceutical compounds or drugs may be encapsulated in drug-polymer coating 340 using a microbead, microparticle or nanoencapsulation technology with albumin, liposome, ferritin or other biodegradable proteins and phospholipids, prior to application on the primer-coated stent.

The bioactive agent may include an antineoplastic agent such as triethylene thiophosphoramide, an antiproliferative agent, an antisense agent, an antiplatelet agent, an antithrombogenic agent, an anticoagulant, an antibiotic, an anti-inflammatory agent, a gene therapy agent, an organic drug, a pharmaceutical compound, a recombinant DNA product, a recombinant RNA product, a collagen, a collagenic derivative, a protein, a protein analog, a saccharide, a saccharide derivative, or combinations thereof.

The bioactive agent is defined as any therapeutic substance that provides a therapeutic characteristic for the prevention and treatment of disease or disorders. An antineoplastic agent may prevent, kill, or block the growth and spread of cancer cells in the vicinity of the stent. An antiproliferative agent may prevent or stop cells from growing. An antisense agent may work at the genetic level to interrupt the process by which disease-causing proteins are produced. An antiplatelet agent may act on blood platelets, inhibiting their function in blood coagulation. An antithrombogenic agent may actively retard blood clot formation. An anticoagulant may delay or prevent blood coagulation with anticoagulant therapy, using compounds such as heparin and coumarins. An antibiotic may kill or inhibit the growth of microorganisms and may be used to combat disease and infection. An anti-inflammatory agent may be used to counteract or reduce inflammation in the vicinity of the stent. A gene therapy agent may be capable of changing the expression of a person's genes to treat, cure or ultimately prevent disease. An organic drug may be any small-molecule therapeutic material. A pharmaceutical compound may be any compound that provides a therapeutic effect. A recombinant DNA product or a recombinant RNA product may include altered DNA or RNA genetic material. Bioactive agents of pharmaceutical value may also include collagen and other proteins, saccharides, and their derivatives.

For example, the bioactive agent may be selected to inhibit vascular restenosis, a condition corresponding to a narrowing or constriction of the diameter of the bodily lumen where the stent is placed. The bioactive agent may generally control cellular proliferation. The control of cell proliferation may include enhancing or inhibiting the growth of targeted cells or cell types. The bioactive agent can be an agent against one or more conditions including coronary restenosis, cardiovascular restenosis, angiographic restenosis, arteriosclerosis, hyperplasia, and other diseases and conditions. For example, the bioactive agent may be selected to inhibit or prevent vascular restenosis, a condition corresponding to a narrowing or constriction of the diameter of the bodily lumen where the stent is placed. The bioactive agent may generally control cellular proliferation. The control of cell proliferation may include enhancing or inhibiting the growth of targeted cells or cell types.

The bioactive agent may include podophyllotoxin, etoposide, camptothecin, a camptothecin analog, mitoxantrone, rapamycin, and their derivatives or analogs. Podophyllotoxin is an organic, highly toxic drug that has antitumor properties and may inhibit DNA synthesis. Etoposide is an antineoplastic that may be derived from a semi-synthetic form of podophyllotoxin to treat monocystic leukemia, lymphoma, small-cell lung cancer, and testicular cancer. Camptothecin is an anticancer drug that may function as a topoisomerase inhibitor. Related in structure to camptothecin, a camptothecin analog such as aminocamptothecin may be used as an anticancer drug. Mitoxantrone is also an important anticancer drug, used to treat leukemia, lymphoma, and breast cancer. Rapamycin or sirolimus is a medication that may interfere with the normal cell growth cycle and may be used to reduce restenosis. The bioactive agent may also include analogs and derivatives of these agents. Antioxidants may be beneficial on their own rights for their antirestonetic properties and therapeutic effects.

Drug-polymer coating 340 may soften, dissolve or erode from the stent to elute at least one bioactive agent. This elution mechanism may be referred to as surface erosion where the outside surface of the drug-polymer coating dissolves, degrades, or is absorbed by the body; or bulk erosion where the bulk of the drug-polymer coating biodegrades to release the bioactive agent. Eroded portions of the drug-polymer coating may be absorbed by the body, metabolized, or otherwise expelled.

The pharmaceutical drug may separate within drug-polymer coating 340 and elute the bioactive agent. Alternatively, the pharmaceutical drug may erode from drug-coated stent 300 and then separate into the bioactive agent. Drug-polymer coating 340 may include multiple pharmaceutical drugs, and may include one or more adhesion promoters. Drug-polymer coating 340 may include a single bioactive agent with optional adhesion promoters to secure the bioactive agent to epoxy primer coating 330 and stent framework 320.

Drug-polymer coating 340 may also include a polymeric matrix. For example, the polymeric matrix may include a caprolactone-based polymer or copolymer, or various cyclic polymers. The polymeric matrix may include various synthetic and non-synthetic or naturally occurring macromolecules and their derivatives. The polymeric matrix may include biodegradable polymers such as polylactide (PLA), polyglycolic acd (PGA) polymer, poly (e-caprolactone) (PCL), polyacrylates, polymethacryates, or other copolymers. The pharmaceutical drug may be dispersed throughout the polymeric matrix. The pharmaceutical drug or the bioactive agent may diffuse out from the polymeric matrix to elute the bioactive agent. The pharmaceutical drug may diffuse out from the polymeric matrix and into the biomaterial surrounding the stent. The bioactive agent may separate from within drug-polymer coating 340 and diffuse out from the polymeric matrix into the surrounding biomaterial.

The polymeric matrix may be selected based on a desired elution rate of the bioactive agent. The pharmaceutical drugs can even be synthesized such that a particular bioactive agent has two different elution rates. A bioactive agent with two different elution rates, for example, would allow rapid delivery of the pharmacologically active drug within twenty-four hours of surgery, with a slower, steady delivery of the drug, for example, over the next two to six months. The epoxy primer coating may be selected to firmly secure the polymeric matrix to the stent framework, the polymeric matrix containing the rapidly deployed bioactive agents and the slowly eluting pharmaceutical drugs.

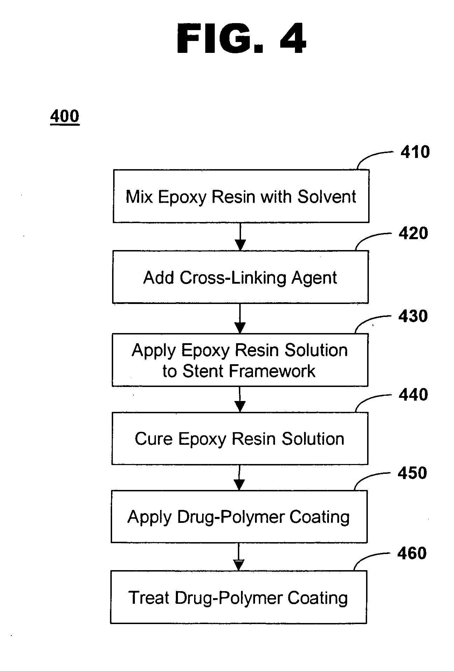

Another aspect of the current invention is a method of manufacturing a coated stent with an epoxy coating or a stent with an epoxy primer coating and a drug-polymer topcoat. FIG. 4 shows a flow diagram of one embodiment of a method for manufacturing an epoxy-coated stent or a drug-coated stent that includes an epoxy primer coating, in accordance with the present invention at 400.

The coated stent with an epoxy coating or epoxy primer coating is manufactured by mixing an epoxy resin with a solvent to form an epoxy resin solution, as seen at block 410. Ketones, esters, solvents such as N-methyl-2-pyrrolidone (NMP) or other suitable epoxy resin solvents may be used. Ketones include acetone, methyl ethyl ketone, methyl iso-butyl or amyl ketone. Esters include ethyl, n-propyl, n-butyl or amyl acetates. The epoxy resin solution may contain, for example, 1-5% epoxy in NMP solvent.

A cross-linking agent may be added to the epoxy resin solution, as seen at block 420. The cross-linking agent may be used to provide additional hardness to the epoxy coating, when additional hardness is desired. During the manufacturing process, the epoxy resin solution may be diluted to fit the coating process. The epoxy resin and curing agent can be premixed at room temperature. The cross-linking agent may be added directly to the epoxy resin solution. Alternatively, the cross-linking agent may be mixed with more of the same type of solvent or with a second solvent that is miscible with the first, and then added to the epoxy resin solution. The epoxy resin and curing agents in their raw form or in solution may be stored separately for longer shelf life.

Polyamine, polyamide, polyacid, polyanhydride or any other suitable agent for cross-linking or curing the epoxy polymer may be used as the cross-linking agent. The cross-linking agent may comprise, for example, 1-5% of the cross-linking agent solution, which may be added into the epoxy resin solution in some predefined ratio, such as 1:1. Alternatively, the cross-linking agent may be mixed in with the epoxy resin solution in a predefined fraction, such as 1-5%.

The epoxy resin solution is applied to a metallic or polymeric stent framework, as seen at block 430. The epoxy resin solution may be applied to the stent framework by dipping, spraying, painting, brushing, or by other suitable methods. Prior to primer application, the stent may be cleaned using, for example, various degreasers, solvents, surfactants and de-ionized water as is known in the art.

The epoxy resin solution disposed on the stent framework is dried and cured, as seen at block 440. Excess liquid may be blown off prior to drying the film. Drying of the polymeric solution to eliminate or remove any volatile components may be done at room temperature or elevated temperatures under a dry nitrogen or other suitable environments including a vacuum environment. During the coating process, the epoxy resin and curing agents may react at room temperature. After coating, the coated stents may be raised to a high temperature to increase the reaction rates between the resin and curing agents. Heating the epoxy resin solution applied to the stent framework to a predetermined curing temperature may cure the epoxy resin solution. The coated stent may be baked at elevated temperatures on the order of 150 to 200 degrees centigrade to drive off any solvent trapped inside the primer coating and to cure the epoxy coating or epoxy primer coating by providing thermal energy for cross linking the epoxy polymer with the cross-linking agent. Full curing of the coating is desired so that solvents used for drug-polymer coatings do not significantly degrade the epoxy coating, and so that high-temperature processing is not needed for drug-polymer applications, which may degrade the drugs.

A second dipping and drying step may be used to thicken the coating when needed. The thickness of the epoxy coating or epoxy primer coating may range between 0.2 microns and 2.0 microns or greater in order to adequately coat and protect the stent framework, and to provide a satisfactory underlayer for subsequent drug-polymer applications. The weight of the epoxy coating or epoxy primer coating depends on the diameter and length of the stent, though a typical weight of the epoxy primer coating is between 20 micrograms and 200 micrograms. Additional application and drying steps may be included to reach the desired thickness of the primer coating and to ensure adequate coverage of the stent framework.

When a drug-polymer coating is desired, the drug-polymer coating is applied to the cured epoxy resin solution disposed on the stent framework, as seen at block 450. The drug polymer may be mixed in a suitable solvent, and applied over the primer using an application technique such as dipping, spraying, painting or brushing. During the coating operation, the drug-polymer adheres well to the epoxy primer coating.

The drug-polymer coating may be applied immediately after the epoxy primer coating is applied. Alternatively, drug-polymer coatings may be applied to a stent with the epoxy primer coating at a later time.

A drug polymer may be mixed with a suitable solvent to form a polymeric solution. The drug polymer may include a polymeric matrix and one or more therapeutic compounds.

To form a drug-polymer coating, a monomer such as a vinyl acetate derivative may be mixed with other monomers in a solvent such as isopropyl alcohol to form a polymeric solution. The mixture may be reacted to form a polymer, and one or more bioactive agents may be mixed with the polymerized mixture to form a drug polymer with a predefined elution rate. A suitable bioactive agent or a solution containing the bioactive agent may be mixed in with the polymeric solution. Alternatively, a polymer such as a copolyester or block copolymer may be dissolved in a suitable solvent, and one or more bioactive agents may be added to the mixture. This mixture may be combined with an adhesion promoter in the polymeric solution. One or more adhesion promoters may be selected and added to the mixture.

The polymeric solution may be applied to the stent framework coated with the epoxy primer. The polymeric solution may be applied to the stent using any suitable method for applying the polymeric solution such as dipping, spraying, painting, or brushing.

Excess liquid may be blown off and the polymeric solution dried. Drying of the polymeric solution to eliminate or remove any volatile components can be done at room temperature or elevated temperatures under a dry nitrogen or other suitable environment such as a vacuum environment. A second dipping and drying step may be used to thicken the coating when needed. The thickness of the drug-polymer coating may range between 1.0 microns and 200 microns or greater in order to provide sufficient and satisfactory pharmacological benefit with the bioactive agent.

The drug-polymer coating may be treated, as seen at block 460. Treatment of the drug-polymer coating may include air drying or low-temperature heating in air, nitrogen, or other controlled environment. The drug-polymer coating may be treated by heating the drug-polymer coating to a predetermined temperature, such as a temperature between 30 and 100 degrees centigrade.

The coated stent with the drug-polymer coating disposed on the epoxy primer coating may be coupled to a catheter. The coated stent may be integrated into a system for treating vascular conditions such as heart disease, by assembling the coated stent onto the catheter. Finished coated stents may be reduced in diameter and placed into the distal end of the catheter, and formed, for example, with an interference fit that secures the stent onto the catheter. The catheter along with the drug-coated stent may be sterilized and placed in a catheter package prior to shipping and storing. Additional sterilization using conventional medical means occurs before clinical use.

Although the present invention applies to cardiovascular and endovascular stents with timed-release pharmaceutical drugs, the use of primer coatings under polymer-drug coatings are applicable to other implantable and blood-contacting biomedical devices such as coated pacemaker leads, microdelivery pumps, feeding and delivery catheters, heart valves, artificial livers and other artificial organs.

## Claims

1. A coated stent, comprising:
a stent framework (220); and
an epoxy coating (230) disposed on the stent framework.

2. A drug-coated stent, comprising:
a stent framework (320);
an epoxy primer coating (330) disposed on the stent framework; and
a drug-polymer coating (340) disposed on the epoxy primer coating.

3. The coated stent of claim 1 or 2 wherein the stent framework comprises a metallic base.

4. The coated stent of claim 3 wherein the metallic base is selected from the group consisting of stainless steel, nitinol, tantalum, MP35N alloy, platinum, titanium, a suitable biocompatible alloy, a suitable biocompatible material, and a combination thereof.

5. The coated stent of any preceding claim wherein the epoxy coating has a thickness between 0.2 and 2.0 microns.

6. The coated stent of any preceding claim wherein the epoxy coating has a weight between 20 and 200 micrograms.

7. The coated stent of any preceding claim wherein the epoxy coating includes a cross-linking agent.

8. The coated stent of claim 7 wherein the cross-linking agent is selected from the group consisting of polyamine, polyamide, polyacid, polyanhydride, and an epoxy curing agent.

9. The drug-coated stent of claim 2 or any claim dependent thereon wherein the stent framework comprises a polymeric base.

10. The drug-coated stent of claim 2 or any claim dependent thereon wherein the drug-polymer coating comprises a bioactive agent.

11. The drug-coated stent of claim 10 wherein the bioactive agent is selected from a group consisting of an antisense agent, an antineoplastic agent, an antiproliferative agent, an antithrombogenic agent, an anticoagulant, an antiplatelet agent, an antibiotic, an anti-inflammatory agent, a gene therapy agent, a therapeutic substance, an organic drug, a pharmaceutical compound, a recombinant DNA product, a recombinant RNA product, a collagen, a collagenic derivative, a protein, a protein analog, a saccharide, and a saccharide derivative.

12. A system for treating a vascular condition, comprising:
a catheter (110);
a stent (120) as claimed in any preceding claim coupled to the catheter.

13. The system of claim 12 wherein the catheter includes a balloon used to expand the stent.

14. The system of claim 12 or 13 wherein the catheter includes a sheath that retracts to allow expansion of the stent.

15. A method of manufacturing a coated stent, comprising:
mixing an epoxy resin with a solvent to form an epoxy resin solution;
adding a cross-linking agent to the epoxy resin solution;
applying the epoxy resin solution onto a stent framework (220) ; and
curing the epoxy resin solution.

16. The method of claim 15 wherein the epoxy resin solution is applied using an application technique selected from the group consisting of dipping, spraying, painting, and brushing.

17. The method of claim 15 or 16 wherein the epoxy resin solution is cured by heating the epoxy resin solution applied to the stent framework to a predetermined curing temperature.

18. The method of any of claims 15 to 17 further comprising;
applying a drug-polymer coating to the cured epoxy resin solution disposed on the stent framework; and
treating the drug-polymer coating.

19. The method of claim 18 wherein the drug-polymer coating is applied using an application technique selected from the group consisting of dipping, spraying, painting, and brushing.

20. The method of claim 18 or 19 wherein the drug-polymer coating is treated by heating the drug-polymer coating to a predetermined drying temperature.
